(19) Europäisches Patentamt
European Patent Office
Office européen des brevets

(11) Publication number: 0 224 386 A2

(12) # EUROPEAN PATENT APPLICATION

(21) Application number: 86309106.2

(51) Int. Cl.[4]: A 61 K 31/565

(22) Date of filing: 20.11.86

(30) Priority: 22.11.85 GB 8528809

(43) Date of publication of application: 03.06.87 Bulletin 87/23

(84) Designated Contracting States: BE CH DE ES FR GB GR IT LI LU NL SE

(71) Applicant: Fink GmbH
Daimlerstrasse 3 Postfach 1160
D-7033 Herrenberg (DE)

(72) Inventor: Wackerle, Lorenz
Kopernikusweg 10
D-8056 Neufahrn (DE)

(74) Representative: Dayneswood, Trevor et al
Beecham Pharmaceuticals Patent and Trade Mark Department Biosciences Research Centre Great Burgh
Yew Tree Bottom Road
Epsom Surrey KT18 5XQ (GB)

The title of the invention has been amended (Guidelines for Examination in the EPO, A-III, 7.3).

(54) **Use of ruscogenins in the treatment of prostate disorders.**

(57) The use of ruscogenin or ruscogenin saponin or Ruscus-extract or an in vivo hydrolysable derivative thereof, (hereinafter referred to as "Ruscogenin") optionally in combination with hesperidine or a chalcone or an in vivo hydrolysable derivative thereof (hereinafter referred to as "Hesperidine")for the manufacture of a medicament for the treatment of prostate disorders, in particular prostatodynia,congestive and obstructive prostatitis. benign prostatic hyperplasia (prostate adenoma), chronic abacterial prostatitis and male adnexitis. Medicaments comprising Ruscogenin and Hesperidine in combination are also novel.

EP 0 224 386 A2

**Description**

Treatment and Composition

The present invention relates to the use of certain steroids in treating prostate disorders.

Ruscogenins are a small class of steroids which can be extracted from Ruscus aculeatus L. (Merck Index 10th Edition 1983 p.8164) and they have hitherto been indicated as being useful for the treatment of hemorrhoids.

It has now been found that Ruscus-extracts (standardised on 3% ruscogenin) are also useful for the treatment of prostate disorders, and in particular prostatodynia, congestive and obstructive prostatitis, benign prostatic hyperplasia (prostate adenoma), chronic abacterial prostatitis and male adnexitis.

According to the present invention there is provided the use of a ruscogenin or a ruscogenin saponin or Ruscus-extract or an in vivo hydrolysable derivative thereof, for the manufacture of a medicament for the treatment of prostate disorders. The term ruscogenin is used herein to include the specific compounds of formulae (I) and (II) disclosed in E. Bombardelli et al. Fito terpia 18, 3, 1972, as well as stereoisomers thereof.

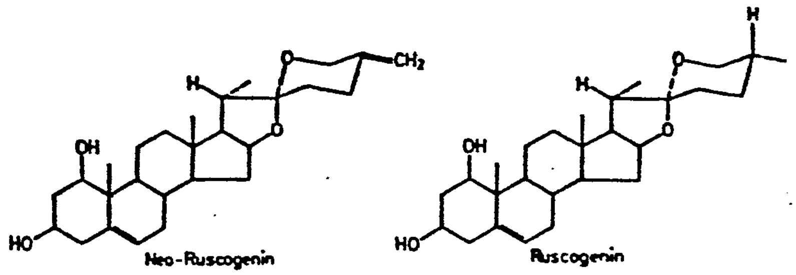

Neo-Ruscogenin

Ruscogenin

Formula (II)

Formula (I)

The term ruscogenin saponin is used herein to include the glycosides of ruscogenin which occur naturally in Ruscus aculeatus L., particularly those designated ruscin, desglucoruscin and desglucodes rhamnoruscin in Bombardelli (supra). The term in vivo hydrolysable derivative is used herein to mean a derivative of ruscogenin or ruscogenin saponin which is hydrolysed in vivo to form the parent compound. For example, one or both of the 1- and 3-hydroxyl groups in formula (I) or (II) may be replaced by labile organic groups connected via ether linkages, such as acyl groups, and the invention encompasses these derivatives when hydrolysable in vivo.

In a preferred aspect of the invention, there is provided the use of a combination of ruscogenin or ruscogenin saponin or Ruscus-extract or derivative thereof as defined above, and hesperidine or a chalcone thereof or an in vivo hydrolysable derivative of hesperidine, for the manufacture of a medicament for the treatment of prostate disorders.

Hesperidine is a flavenoid, and is preferably used in the form of a hesperidine methyl chalcone derivative of formula (III)

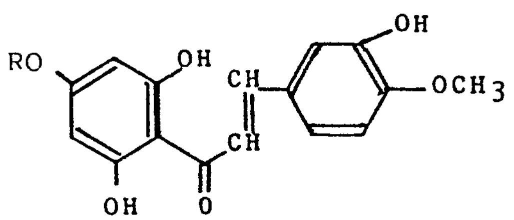

(III)

in which R is a rhamnosyl glucose group, or trimethyl hesperidine chalcone of formula (IV)

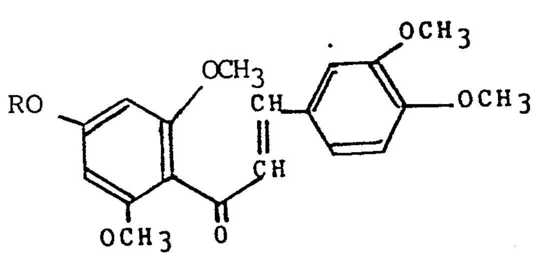

(IV)

in which R is as defined in formula (III).

For ease of reference, the term "Ruscogenin" will be used hereinafter to mean ruscogenin per se, ruscogenin saponin, Ruscus-extract or an in vivo hydrolysable derivative of any thereof, and the term "Hesperidine" will be used to mean hesperidine per se, a chalcone thereof or an in vivo hydrolysable derivative thereof.

In a further aspect of the invention there is provided a medicament comprising Ruscogenin and Hesperidine in combination with a pharmaceutically acceptable diluent or excipient.

The medicament for the treatment of prostate disorders contains pharmaceutically effective amounts of Ruscogenin and, optionally, Hesperidine, in combination with a pharmaceutically acceptable diluent or excipient.

Preferably, the medicament comprises from 0.003 to 2.1% by weight of Ruscogenin, and optionally from 0.1 to 50% by weight of Hesperidine, based on the total weight of the medicament.

Particularly preferred amounts of Ruscogenin are from 0.45 to 1.35% by weight, and those of Hesperidine from 15 to 30% by weight.

The preferred weight ratio of a Ruscus-extract (standardised on 3% ruscogenin) to Hesperidine in the medicament is from about 0.5 : 1 to 2:1, particularly about 0.8:1 to l:2:l.
to l.2:1.

The medicament may be formulated for administration by the oral, parenteral or rectal route, and may be in the form of tablets, capsules, powders, granules, lozenges or liquid preparations such as oral or sterile parenteral solutions or suspensions.

Tablets and capsules for administration may be in unit dose presentation form, and may contain conventional excipients such as binding agents, for example syrup, acacia, gelatin, sorbitol, tragacanth, or polyvinylpyrollidone: fillers, for example lactose, sugar, maize-starch, calcium phosphate, sorbitol or glycine, tabletting for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Capsules may be in the form of hard gelatin or soft gelatin capsules, preferably the latter.

Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol, preservatives, for example methyl or propyl p-hydroxybenzoate or sorbic acid, and, if desired, conventional flavouring or colouring agents.

Medicaments for administration by the rectal route are preferably in the form of suppositories, which will contain conventional suppository bases, e.g. cocoa-butter, hardened vegetable fat, triglycerides, polyethylene glycols, plus fatty alcohols, silicon dioxide, stearates or lecithin.

The oral and rectal presentation forms described above may be made in accordance with conventional pharmaceutical techniques.

For parenteral administration, fluid unit dosage forms are prepared utilizing a sterile vehicle, water being preferred. The active ingredients depending on the vehicle and concentration used, can be either suspended or disssolved in the vehicle. In preparing solutions the ingredients can be dissolved in water for injection and filter sterilised before filling into a suitable vial or ampoule and sealing. Advantageously, agents such as a local anaesthetic, preservative and buffering agents can be dissolved in the vehicle. To enhance the stability, the medicament can be frozen after filling into the vial and the water removed under vacuum. The dry lyophilized powder is then sealed in the vial and an accompanying vial of water for injection may be supplied to reconstitute the liquid prior to use. Parental suspensions are prepared in substantially the same manner except that the ingredients are suspended in the vehicle instead of being dissolved and sterilisation cannot be accomplished by filtration. The ingredients can be sterilised by exposure to ethylene oxide before suspending in the sterile vehicle. Advantageously, a surfactant or wetting agent is included in the medicament to facilitate uniform distribution of the ingredients.

The parental presentation forms described above may be made in accordance with conventional pharmaceutical techniques.

Where the medicament comprises dosage units, each unit will preferably contain from 35 to 325mg of each of the active ingredients. The dosage as employed for adult human treatment will preferably range from 35 to 2100mg of each active ingredient per day, for instance 350mg per day depending on the route and frequency of administration. Such a dosage corresponds to 0.5 to 5mg/kg per day. Suitably the dosage is from 0.5 to 30mg/kg per day.

The invention is illustrated by means of the following Examples, which are medicaments for use in treating prostate disorders.

Example I

A tablet, made by conventional tabletting techniques has the following composition:

| | |
|---|---|
| Ruscus-extract (standardised on 4·5mg ruscogenin) | 150mg |
| Hesperidine chalcone | 75mg |
| Talcum | 250mg |
| Lactose | 20mg |
| Magnesium stearate | 5mg |
| | 500mg |

Example 2

A conventionally produced soft gelatine capsule has the following composition:

| | |
|---|---|
| Hesperidine chalcone | 35mg |
| Ruscus-extract (standardised on 2·1 mg ruscogenin) | 70mg |
| Soyalecithin | 10mg |
| Bee wax | 31mg |
| Soyabean oil partially hydrogenated | 50mg |
| Gelatine coat | 251mg |
| | 447mg |

**Claims**

I. The use of ruscogenin or ruscogenin saponin or Ruscus-extract or an in vivo hydrolysable derivative thereof (hereinafter referred to as "Ruscogenin") for the manufacture of a medicament for the treatment of prostate disorders.

2. The use according to claim I, wherein the Ruscogenin comprises a compound of formula I or II or a stereoisomer thereof:

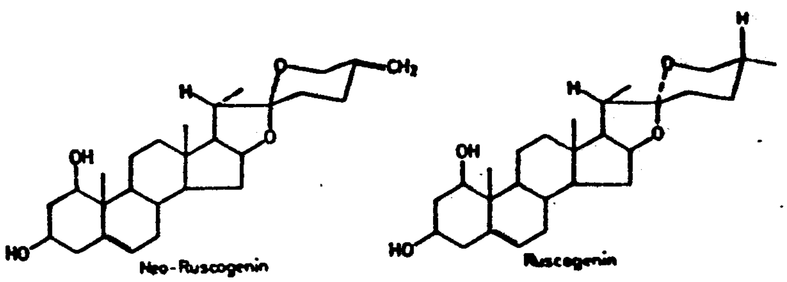

Neo-Ruscogenin Ruscogenin

3. The use according to claim I or 2, wherein the medicament comprises from 0.003 to 2.I% by weight of Ruscogenin based on the total weight of the medicament, in combination with a pharmaceutically acceptable diluent or excipient.

4. The use according to claim 3, wherein the medicament comprises from 0.45 to I.35% by weight of Ruscogenin based on the total weight of the medicament.

5. The use according to anyone of claims I to 4, wherein the Ruscogenin is used in combination with hesperidine or a chalcone thereof or an <u>in vivo</u> hydrolysable derivative thereof, (hereinafter referred to as "Hesperidine").

6. The use according to claim 5, wherein the Hesperidine is used in the form of an hesperidine methyl chalcone derivative of formula III or a trimethyl hesperidine chalcone of formula IV:

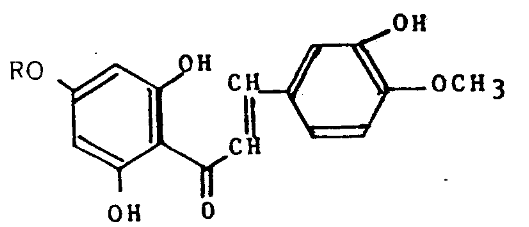

(III)

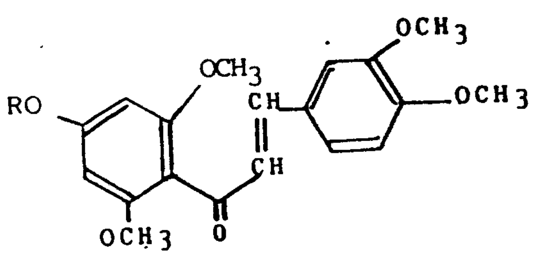

(IV)

in which formulae R is a rhamnosyl glucose group. 7. The use according to claim 5 or 6, wherein the medicament comprises from 0.I to 50% by weight of Hesperidine based on the total weight of medicament in combination with a pharmaceutically acceptable diluent or excipient.

8. The use according to claim 7, wherein the medicament comprises from I5 to 30% by weignt of Hesperidine based on the total weight of the medicament.

9. The use according to any one of claims 5 to 8, wherein the medicament comprises the Ruscogenin and Hesperidine in a weight ratio of from 0.5:I to 2:I respectively

I0. The use according to any one of claims 5 to 9, wherein the medicament is in unit dosage form and comprises from 35 to 325 mg of each of Ruscogenin and Hesperidine, per unit dosage.

II. The use according to any one of claims I to I0, wherein the medicament is for the treatment of prostatodynia, congestive or obstructive prostatitis, benign prostatic hyperplasia, chronic abacterial prostatitis, or male adnexitis.

I2. A medicament, comprising ruscogenin or rusogenin saponin or Ruscus-extract or an <u>in vivo</u> hydrolysable derivative thereof (hereinafter referred to as "Ruscogenin") and hesperidine or a chalcone thereof or an <u>in vivo</u> hydrolysable derivative thereof (hereinafter referred to as "Hesperidine"), in combination with a pharmaceutically acceptable diluent or excipient.

I3. A medicament according to claim I2 wherein the Ruscogenin comprises a compound of formula I or II

or a stereoisomer thereof:

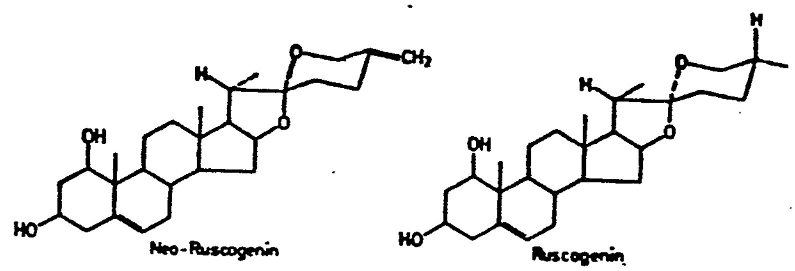

Neo-Ruscogenin Ruscogenin

(I) (II)

14. A medicament according to claim 12 or 13 wherein the medicament comprises from 0.003 to 2.1% by weight weight of Ruscogenin based on the total weight of the medicament.

15. A medicament according to claim 14 wherein the medicament comprises from 0.45 to 1.35% by weight of Ruscogenin based on the total weight of the medicament.

16. A medicament according to any one of claims 12 to 15 wherein the Hesperidine is used in the form of an hesperidine methyl chalcone derivative of formula III or a trimethyl hesperidine chalcone of formula IV:

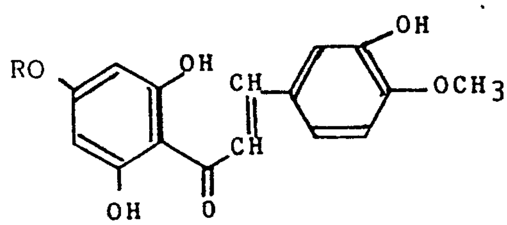

(III)

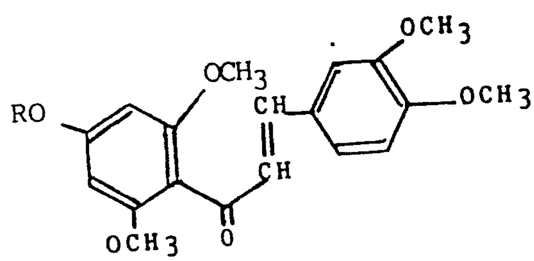

(IV)

17. A medicament according to any one of claims 12 to 16, wherein the medicament comprises from 0.1 to 50% by weight of Hesperidine based on the total weight of medicament.

18. A medicament according to claim 17. wherein the medicament comprises from 15 to 30% by weight of Hesperidine based on the total weight of the medicament.

19. A medicament according to any one of claims 12 to 18, wherein the medicament comprises the Ruscogenin and Hesperidine in a weight ratio of from 0.5:1 to 2:1 respectively.

20. A medicament according to any one of claims 12 to 20, wherein the medicament is in unit dosage form and comprises from 35 to 325 mg of each of Ruscogenin and Hesperidine, per unit dosage.